# EUROPÄISCHE PATENTSCHRIFT

(11) **EP 1 193 223 B1**
(45) Veröffentlichungstag und Bekanntmachung des Hinweises auf die Patenterteilung: **12.05.2004**
(21) Anmeldenummer: 01122959.8
(22) Anmeldetag: 25.09.2001
(51) Int. Cl.: C02F 11/18, C02F 1/02

(54) **Verfahren und Vorrichtung zur Behandlung tierischer Nebenprodukte**
Process and apparatus for the treatment of animal by-products
Procédé et dispositif pour le traitement de sous-produits d'animaux

(30) Priorität: 02.10.2000 DE 10048828; 23.01.2001 DE 10117321
(43) Veröffentlichungstag der Anmeldung: 03.04.2002
(73) Patentinhaber: ATZ-EVUS Applikations- und Technikzentrum für Energieverfahrens-, Umwelt- und Strömungstechnik, 92237 Sulzbach-Rosenberg (DE)
(72) Erfinder: Dimaczek, Gerold, Dipl.-Ing., 91052 Erlangen (DE); Schneider, Ralf, Dipl.-Ing., 91207 Lauf (DE)
(74) Vertreter: Gassner, Wolfgang, Dr.

(56) Entgegenhaltungen:
- EP-A- 0 646 547
- WO-A-99/04897
- WO-A-99/47250
- US-A- 5 888 307

## Beschreibung

Die Erfindung betrifft ein Verfahren und eine Vorrichtung zur Behandlung von biogenen Restmassen bzw. organischen Abfällen, wie Tierkadavern und dgl..

Nach dem Stand der Technik ist aus der DE 689 05 362 T2 ein Verfahren zur kontinuierlichen Behandlung von totem, tierischen Material bekannt. Dabei wird das Material kontinuierlich durch eine verhältnismäßig lange fortlaufende Röhre geführt. In der Röhre wird es bei einer Temperatur von höchstens 140°C sterilisiert. Das Vorsehen einer verhältnismäßig langen fortlaufenden Röhre ist aufwendig. Außerdem kann bei dem bekannten Verfahren nicht immer sichergestellt werden, daß das Material vollständig zersetzt wird.

Ein weiteres Verfahren zur kontinuierlichen Behandlung organischer Restmassen ist aus der WO 99/04897 bekannt. Dabei wird die organische Restmasse in einem Schlaufenreaktor erhöhter Temperatur und erhöhtem Druck ausgesetzt. Auch bei diesem kontinuierlichen Verfahren kann nicht immer sichergestellt werden, daß die organische Restmasse durch die im Schlaufenreaktor stattfindende Temperatur-Druck-Hydrolyse vollständig zersetzt wird. Es kommt hier mitunter vor, daß teilweise unzersetztes Material den Schlaufenreaktor verläßt.

Aufgabe der Erfindung ist es, die Nachteile nach dem Stand der Technik zu beseitigen. Es sollen insbesondere ein Verfahren und eine Vorrichtung angegeben werden, mit denen biogene Restmassen zuverlässig vollständig abgebaut werden können. Weiteres Ziel ist es, das Verfahren und die Vorrichtung möglichst effizient, kostengünstig und einfach auszuführen.

Diese Aufgabe wird durch die Merkmale der Ansprüche 1 und 11 gelöst. Zweckmäßige Ausgestaltungen ergeben sich aus den Merkmalen der Ansprüche 2 bis 10 und 12 bis 20.

Mit dem erfindungsgemäßen Verfahren sowie der Vorrichtung wird sichergestellt, daß die biogene Restmasse stets ausreichend lange der Temperatur-Druck-Hydrolyse ausgesetzt wird. Das Verfahren wird nicht kontinuierlich, sondern quasi-kontinuierlich geführt. Durch das Vorsehen mehrerer erster Entspannungsbehälter ist es möglich, die Hydrolyselösung im Reaktor für eine ausreichend lange vorgegebene Zeit zu halten und dann erst in einen der ausgewählten Entspannungsbehälter zu entlassen. Die vorgeschlagene Vorrichtung ist relativ einfach aufgebaut. Sie erfordert nicht das kostenaufwendige und einen hohen Platzbedarf benötigende Vorsehen einer langen fortlaufenden Röhre. Außerdem können gesetzlich vorgegebene Haltezeiten in den Entspannungsbehältern sicher eingehalten werden.

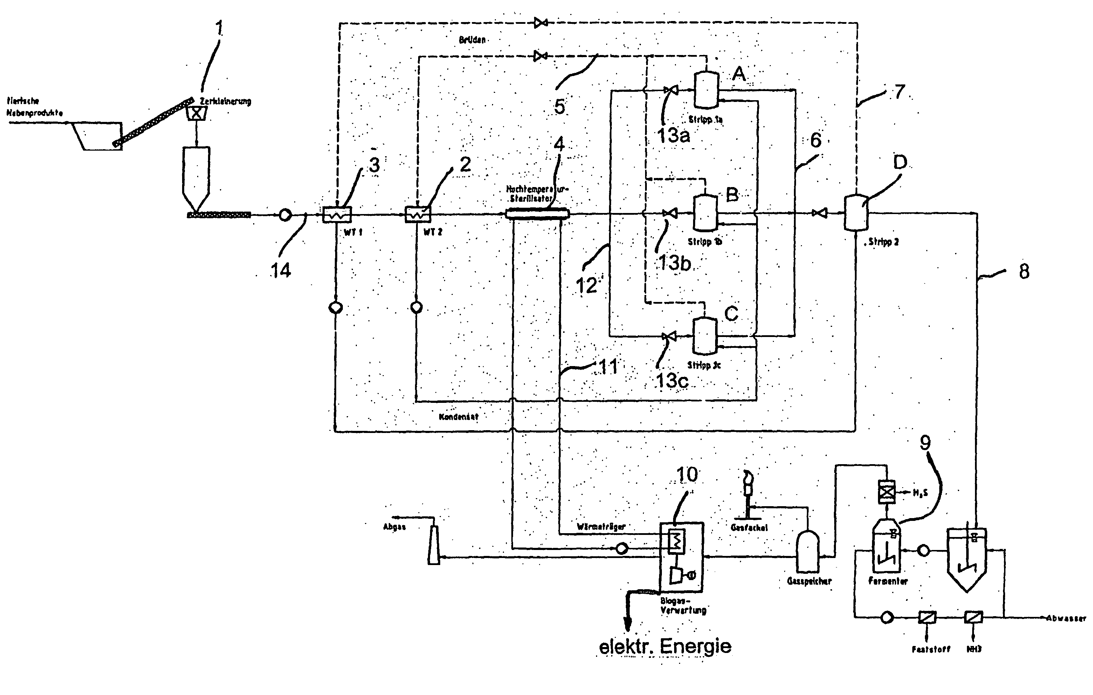

Nachfolgend wird ein Ausführungsbeispiel der Vorrichtung anhand der einzigen Figur näher erläutert. Die Figur zeigt schematisch den Aufbau einer erfindungsgemäßen Vorrichtung.

Mit 1 ist eine Zerkleinerungsvorrichtung bezeichnet, in der die aufgegebene biologische Restmasse zerkleinert wird. Die zerkleinerte biologische Restmasse kann nötigenfalls mit einer Flüssigkeit, z.B. Wasser, biologischen flüssigen Abfällen und dgl., verflüssigt werden. Die verflüssigte biogene Restmasse kann dann mittels Pumpen über eine Zuführleitung 14 transportiert werden. Sie führt über einen ersten 2 und einen zweiten Wärmetauscher 3 zu einem Reaktor bzw. Hochtemperatursterilisator 4. Der Hochtemperatursterilisator 4 kann als Rohrreaktor, wie z.B. in der DE 689 05 362 T2 offenbart, ausgebildet sein. Dem Hochtemperatursterilisator 4 sind stromabwärts drei erste Entspannungsbehälter A, B und C in paralleler Anordnung nachgeschaltet. Die drei ersten Entspannungsbehälter A, B und C sind wahlweise mit dem Hochtemperatursterilisator 4 verbindbar.

Der Hochtemperatursterilisator 4 ist über eine verzweigte Leitung 12 mit den drei ersten Entspannungsbehältern A, B und C verbunden. In jeden Leitungsast der verzweigten Leitung 12 ist ein Ventil 13a, 13b und 13c eingeschaltet. Durch eine geeignete automatische Steuerung der Ventile kann die aus dem Hochtemperatursterilisator 4 austretende Hydrolyselösung in einen der ersten Entspannungsbehälter A, B oder C überführt werden.

Die ersten Entspannungsbehälter A, B und C sind über eine erste Ringleitung 5 mit dem ersten Wärmetauscher 2 verbunden. Ferner sind die drei. ersten Entspannungsbehälter A, B und C über eine erste Leitung 6 mit einem stromabwärts nachgeschalteten zweiten Entspannungsbehälter D verbunden. Der zweite Entspannungsbehälter D ist über eine zweite Ringleitung 7 mit dem zweiten Wärmetauscher 3 verbunden. Vom zweiten Entspannüngsbehalter D führt eine zweite Leitung 8 zu einem Fermenter 9 und einer stromabwärts nachgeschalteten Biogasverwertungseinrichtung 10. Aus der Biogasverwertungseinrichtung 10 ausgekoppelte Wärme wird mittels eines Wärmeträgermediums über eine dritte Leitung 11 dem Hochtemperatursterilisator 4 zugeführt.

Die Funktion der Vorrichtung ist folgende:

Zunächst wird der organische Abfall bzw. die biogene Restmasse, wie Tierkadaver und dgl., in der Zerkleinerungsvorrichtung 1 zerkleinert und nötigenfalls verflüssigt. Die fließfähige biogene Restmasse wird über die Zuführleitung 14 zum Hochtemperatursterilisator 4 gepumpt. Sie wird durch den in die Zuführleitung 14 eingeschalteten ersten 2 sowie zweiten Wärmetauscher 3 vorgewärmt. Im Hochtemperatursterilisator 4 wird die biogene Restmasse auf eine Temperatur von 150 bis 250° C erhitzt. Gleichzeitig wird ein Druck von 10 bis 50 bar aufgebracht. Die biogene Restmasse wird im Hochtemperatursterilisator 4 für eine vorgegebene Haltezeit, z.B. 20 Minuten, gehalten. Anschließend wird über eine automatische Steuerung z.B. das erste Ventil 13a geöffnet. Die im Hochtemperatursterilisator 4 gebildete Hydrolyselösung wird in den ersten Entspannungsbehälter A überführt. Bei der Überführung wird die Hydrolyselösung gekühlt und entspannt. Die bei der Entspannung entstehenden Dämpfe, die sogenannten Brüden, werden über die Ringleitung 5 zum ersten Wärmetauscher 2 geleitet. Das im ersten Wärmetauscher 2 gebildete Kondensat wird über die Ringleitung 5 zum ersten Entspannungsbehälter A zurückgeführt.

Gleichzeitig wird z.B. im weiteren ersten Entspannungsbehälter B eine zuvor überführte Hydrolyselösung für eine vorgegebene Haltezeit, z.B. 20 Minuten, auf einer Temperatur von mehr als 133 °C und einem Druck von mehr als 3 bar gehalten.

Gleichzeitig kann aus dem in diesem Beispiel Verbliebenen weiteren ersten Entspannungsbehälter C eine darin befindliche teilweise entspannte und abgekühlte Hydrolyselösung über die erste Leitung in den zweiten Entspannungsbehälter D überführt werden. Die Hydrolyselösung wird dabei auf Umgebungsdruck entspannt. Die dabei entstehenden weiteren Brüden werden über die zweite Ringleitung zum zweiten Wärmetauscher 3 geleitet. Das dort entstehende weitere Kondensat wird wiederum über die zweite Ringleitung 7 zum zweiten Entspannungsbehälter D zurückgeführt. Die Temperatur des weiteren Kondensats beträgt dann etwa 100 °C.

Die Dauer eines Zykluses wird im wesentlichen durch die Haltezeit in den ersten Entspannungsbehältern A, B und C bestimmt. Nach Beendigung eines Zykluses, d.h. nach Ablauf der Haltezeit von z.B. 20 Minuten im ersten Entspannungsbehälter B, wird der erste Entspannungsbehälter A für eine Zeit von mindestens 20 Minuten auf einer Temperatur auf mehr als 133 °C und einem Druck von mehr als 3 bar gehalten. Gleichzeitig wird vom ersten Entspannungsbehälter B die teilweise entspannte Hydrolyselösung in den zweiten Entspannungsbehälter D überführt und dort weiter abgekühlt. Währenddessen wird der weitere erste Entspannungsbehälter C mit von dem Höchtemperatursterilisator 4 zugeführter Hydrolyselösung befüllt.

Nach Beendigung dieses weiteren Zykluses wird die teilentspannte Hydrolyselösung vom ersten Entspannungsbehälter A in den zweiten Eintspannungsbehälter D überführt. Der erste Entspahnungsbehälter B wird währenddessen gefüllt, im weiteren ersten Entspannungsbehälter C wird die Hydrolyselösung unter den vorbeschriebenen Temperatur- und Druckbedingungen gehalten.

Die entspannte Hydrolyselösung wird dann über die zweite Leitung 8 dem Fermenter zugeführt und mittels Mikroorganismen, wie z.B. anaeroben Bakterien, umgesetzt. Bei der Umsetzung anfallendes Biogas wird in der Biogasverwertungseinrichtung 10 u.a. zur Auskopplung von Wärme genutzt, welche teilweise über die dritte Leitung 11 dem Hochtemperatursterilisator 4 zur Verfügung gestellt wird. Selbstverständlich können anstatt Biogas auch andere Wertstoffe aus der entspannten Hydrolyselösung mittels Mikroorganismen gewonnen werden.

Das Verfahren wird quasi-kontinuierlich geführt. Jeder der drei ersten Entspannungsbehälter A, B und C kann wechselweise zur Durchführung des Befüll-, Halte- oder Entspannungsschritts benutzt werden. Wenn einer der ersten Entspannungsbehälter A, B oder C in den zweiten Entspannungsbehälter entleert wird, kann gleichzeitig ein anderer der ersten Entspannungsbehälter A, B oder C mit vom Hochtemperatursterilisator 4 kommender Hydrolyselösung befüllt werden. Wiederum ein anderer der drei ersten Entspannungsbehälter A, B oder C kann gleichzeitig bei einer Temperatur von mehr als 133 °C und einem Druck von mehr als 3 bar gehalten werden. Auf diese Weise kann die biogene Restmasse z.B. mit einer Taktzeit von 20 Minuten abgebaut werden. Das Vorsehen einer solchen Taktzeit gewährleistet jederzeit, daß die Temperatur, der Druck und die Zeitvorgaben zur vollständigen Sterilisation gemäß dem Tierkörperbeseitigungsgesetz erfüllt werden.

### Bezugszeichenliste

- 1: Zerkleinerungsvorrichtung
- 2: erster Wärmetauscher
- 3: zweiter Wärmetauscher
- 4: Hochtemperatursterilisator
- 5: erste Ringleitung
- 6: erste Leitung
- 7: zweite Ringleitung
- 8: zweite Leitung
- 9: Fermenter
- 10: Biogasverwertungseinrichtung
- 11: dritte Leitung
- 12: verzweigte Leitung
- 13a,b,c: Ventile
- 14: Zuführungsleitung
- A, B, C: erste Entspannungsbehälter
- D: zweiter Entspannungsbehälter

## Patentansprüche

1. Verfahren zur quasi-kontinuierlichen Behandlung von biogenen Restmassen, wie Tierkadavern und dgl., mit folgenden Schritten:
a) Zerkleinern der biogenen Restmassen,
b) Erhitzen der biogenen Restmassen für eine vorgegebene Haltezeit auf eine Temperatur im Bereich von 150°C bis 250°C und Aufbringen eines Drucks von 10 bis 50 bar, so dass eine Hydrolyselösung gebildet wird,
c) wechselweise Auswahl eines von mehreren ersten Entspannungsbehältern (A, B, C) und Überführen der Hydrolyselösung in den ausgewählten ersten Entspannungsbehälter (A, B, C),
d) Abkühlen und Entspannen der Hydrolyselösung und
e) Umsetzen der Hydrolyselösung mittels Mikroorganismen.

2. Verfahren nach Anspruch 1, Wobei die Hydrolyselösung beim Schritt lit.c in einem der ersten Entspannungsbehälter (A, B, C) für mindestens 20 Minuten auf einer Temperatur von mehr als 133°C und einem Druck von mehr als 3 bar gehalten wird.

3. Verfahren nach Anspruch 2, wobei die Hydrolyselösung vom ersten (A, B, C) in einen weiteren ersten Entspannungsbehälter (A, B, C) überführt wird.

4. Verfahren nach einem der vorhergehenden Ansprüche, wobei beim Entspannen der Hydrolyselösung im ersten Entspannungsbehälter (A, B, C) gebildete Brüden zum Vorwärmen der biogenen Restmasse über einen ersten Wärmetauscher (2) geführt werden.

5. Verfahren nach einem der vorhergehenden Ansprüche, wobei drei erste Entspannungsbehälter (A, B, C) vorgesehen sind und wobei während des Haltens der Temperatur im einen der ersten Entspannungsbehälter (A, B, C) einer der anderen ersten Entspannungsbehälter geleert und ein weiterer der anderen ersten Entspannungsbehälter (A, B, C) befüllt wird.

6. Verfahren nach einem der Ansprüche 4 oder 5, wobei das im ersten Wärmetauscher (2) aus den Brüden gebildete Kondensat in den in Befüllung befindlichen ersten Entspannüngsbehälter (A, B, C) zurückgeführt wird

7. Verfahren nach einem der vorhergehenden Ansprüche, wobei die Hydrolyselösung in mindestens einen den ersten Entspannungsbehältern (A, B, C) stromabwärts nachgeschalteten zweiten Entspannungsbehälter (D) überführt wird.

8. Verfahren nach Anspruch 7, wobei beim Entspannen der Hydrolyselösung im zweiten Entspannungsbehälter (D) gebildete weitere Brüden zum Vorwärmen der biogenen Restmasse über einen zweiten Wärmetauscher (3) geführt werden.

9. Verfahren nach Anspruch 8, wobei das im zweiten Wärmetauscher (3) aus den weiteren Brüden gebildete weitere Kondensat in den zweiten Entspannungsbehälter (D) zurückgeführt wird

10. Verfahren nach einem der vorhergehenden Ansprüche, wobei die biogene Restmasse beim Schritt lit. b durch einen Schlaufenreaktor geführt wird.

11. Vorrichtung zur Durchführung des Verfahrens nach einem der vorhergehenden Ansprüche, umfassend
a) ein Mittel (1) zum Zerkleinern von biogener Restmasse,
b) ein Mittel (4) zum Erhitzen der biogenen Restmassen auf eine Temperatur im Bereich von 150°C bis 250°C und zum Aufbringen eines Drucks von 10 bis 50 bar, so dass eine Hydrolyselösung gebildet wird,
c) ein Mittel zur wechselweisen Auswahl eines von mehreren ersten Entspannungsbehältern (A, B, C) und ein Mittel (12, 13a, 13b, 13c) zum Überführen der Hydrolyselösung in den ausgewählten Entspannungsbehälter (A, B, C),
d) ein Mittel (D) zum Abkühlen und Entspannen der Hydrolyselösung und
e) ein Mittel (10) zur Umsetzung der Hydrolyselösung mittels Mikroorganismen.

12. Vorrichtung nach Anspruch 11, wobei das Mittel (4) zum Erhitzen der biogenen Restmassen auf eine Temperatur im Bereich von 150 °C bis 250 °C und zum Aufbringen eines Drucks von 10 bis 50 bar einen Schlaufenreaktor umfaßt.

13. Vorrichtung nach Anspruch 11 oder 12, wobei ein Mittel (12) zum Überführen der Hydrolyselösung vom ersten (A, B, C) in einen weiteren ersten Entspannungsbehälter (A, B, C) vorgesehen ist.

14. Vorrichtung nach einem der Ansprüche 11 bis 13, wobei ein mit den ersten Entspannungsbehälteren (A, B, C) verbundener erster Wärmetauscher (2) zum Vorwärmen der biogenen Restmasse vorgesehen ist.

15. Vorrichtung nach einem der Ansprüche 11 bis 14, wobei drei erste Entspannungsbehälter (A, B, C) vorgesehen sind.

16. Vorrichtung nach einem der Ansprüche 11 bis 15, wobei den ersten Entspannungsbehältern (A, B, C) mindestens ein stromabwärts nachgeschalteter zweiter Entspannungsbehälter (D) vorgesehen ist.

17. Vorrichtung nach einem der Ansprüche 11 bis 16, wobei der zweite Entspannungsbehälter (D) mit einem zweiten Wärmetauscher (3) zum Vorwärmen der biogenen Restmasse verbunden ist.

18. Vorrichtung nach einem der vorhergehenden Ansprüche 11 bis 17, wobei zur Rückführung des im ersten Wärmetauscher (2) aus den Brüden gebildeten Kondensats eine den ersten Wärmetauscher (2) mit den ersten Entspannungsbehältern (A, B, C) verbindende erste Ringleitung (5) vorgesehen ist.

19. Vorrichtung nach einem der vorhergehenden Ansprüche 11 bis 18, wobei zur Rückführung des im zweiten Wärmetauscher (3) aus den weiteren Brüden gebildeten weiteren Kondensats eine den zweiten Wärmetauscher (3) mit dem zweiten Entspannungsbehälter (D) verbindende zweite Ringleitung (7) vorgesehen ist.

20. Vorrichtung nach einem der Ansprüche 11 bis 19, wobei die biogene Restmasse beim Schritt lit. b durch einen Schlaufenreaktor geführt wird.

## Claims

1. Method for pseudo-continuous treatment of residual biogenous masses such as animal cadavers and similar consisting of the following steps:
a) Chopping up the residual biogenous masses,
b) heating of the residual biogenous masses for a specified holding time to a temperature in the range from 150 °C to 250 °C and application of a pressure of 10 to 50 bar so that a hydrolysis solution is created,
c) alternating selection of one of several first expansion tanks (A, B, C) and transfer of the hydrolysis solution to the selected first expansion tank (A, B, C),
d) cooling off and expanding the hydrolysis solution and
e) conversion of the hydrolysis solution by means of micro-organisms.

2. Method as defined in claim 1, wherein the hydrolysis solution is held during step c in one of the first expansion tanks (A, B, C) for at least 20 minutes at a temperature of more than 133 °C and a pressure of more than 3 bar.

3. Method as defined in claim 2, wherein the hydrolysis solution is transferred from the first (A, B, C) to a further first expansion tank (A, B, C).

4. Method as defined in one of the preceding claims, wherein the vapors created during expansion of the hydrolysis solution in the first expansion tank (A, B, C) are transferred for prewarming of the residual biogenous mass via a first heat exchanger (2).

5. Method as defined in one of the preceding claims, wherein three first expansion tanks (A, B, C) are provided and, wherein, while the temperature is being held in one of the first expansion tanks (A, B, C), one of the other first expansion tanks is being emptied and another of the other first expansion tanks (A, B, C) is being filled.

6. Method as defined in one of the claims 4 or 5, wherein the condensate created from the vapors in the first heat exchanger (2) is returned to the first expansion tank (A, B, C) which is being filled.

7. Method as defined in one of the preceding claims, wherein the hydrolysis solution is transferred to at least one second expansion tank (D) located downcurrent after the first expansion tanks (A, B, C).

8. Method as defined in claim 7, wherein the further vapors created during expansion of the hydrolysis solution in the second expansion tank (D) are transferred for prewarming the residual biogenous mass via a second heat exchanger (3).

9. Method as defined in claim 8, wherein the further condensate created from the further vapors in the second heat exchanger (3) is returned to the second expansion tank (D).

10. Method as defined in one of the preceding claims, wherein the residual biogenous mass is fed during step b through a loop-type reactor.

11. Device for execution of the method as defined in one of the preceding claims, comprising
a) a means (1) of chopping up residual biogenous mass,
b) a means (4) of heating the residual biogenous masses to a temperature in the range of 150 °C to 250 °C and for application of a pressure of 10 to 50 bar so that a hydrolysis solution is created,
c) a means of alternating selection of one of several first expansion tanks (A, B, C) and a means (12, 13a, 13b, 13c) of transferring the hydrolysis solution to the selected expansion tank (A, B, C),
d) a means (D) of cooling off and expanding the hydrolysis solution and
e) a means (10) of converting the hydrolysis solution by means of micro-organisms.

12. Device as defined in claim 11, wherein the means (4) of heating the residual biogenous masses to a temperature in the range from 150 °C to 250 °C and to apply a pressure of 10 to 50 bar includes a loop-type reactor.

13. Device as defined in claim 11 or 12, wherein a means (12) of transferring the hydrolysis solution from the first (A, B, C) to a further first expansion tank (A, B, C) is provided.

14. Device as defined in one of the claims 11 to 13, wherein a first heat exchanger (2) connected with the first expansion tanks (A, B, C) for preheating the residual biogenous mass is provided.

15. Device as defined in one of the claims 11 to 14, wherein three first expansion tanks (A, B, C) are provided.

16. Device as defined in one of the claims 11 to 15, wherein at least one second expansion tank (D) is provided positioned downcurrent after the first expansion tanks (A, B, C).

17. Device as defined in one of the claims 11 to 16, wherein the second expansion tank (D) is connected with a second heat exchanger (3) for preheating the residual biogenous mass.

18. Device as defined in one of the preceding claims 11 to 17, wherein a first ring line (5) connecting the first heat exchanger (2) with the first expansion tanks (A, B, C) is provided for returning the condensate created from the vapors in the first heat exchanger (2).

19. Device as defined in one of the preceding claims 11 to 18, wherein a second ring line (7) connecting the second heat exchanger (3) with the second expansion tank (D) is provided to return the additional condensate created from the further vapors in the second heat exchanger (3).

20. Device as defined in one of the claims 11 to 19, wherein the residual biogenous mass is fed during step b through a loop-type reactor.

## Revendications

1. Procédé pour le traitement quasi continu de masses résiduelles biogènes, telles que des cadavres animaux et id., en effectuant les opérations suivantes:
a) broyage des masses résiduelles biogènes,
b) chauffage des masses résiduelles biogènes pour un temps de maintien défini à une température comprise dans une plage de 150°C à 250°C et à application d'une pression de 10 à 50 bar de façon à former une solution d'hydrolyse,
c) sélection alternative d'une de plusieurs premières cuves de détente (A, B, C) et transport de la solution d'hydrolyse dans la première cuve de détente sélectionnée (A, B, C),
d) refroidissement et détente de la solution d'hydrolyse et
e) transformation de la solution d'hydrolyse au moyen de microorganismes.

2. Procédé selon la revendication 1, la solution à l'opération mentionnée au point c) étant maintenue dans une des premières cuves de détente (A, B, C) pour au moins 20 minutes à une température supérieure à 133°C et à une pression supérieure à 3 bar.

3. Procédé selon la revendication 2, la solution d'hydrolyse étant transportée de la première (A, B, C) à une autre première cuve de détente (A, B, C).

4. Procédé selon l'une des revendications précédentes, les vapeurs formées lors de la détente de la solution d'hydrolyse dans la première cuve de détente (A, B, C) pour préchauffer la masse résiduelle biogène étant conduites via un premier échangeur thermique (2).

5. Procédé selon l'une des revendications précédentes, trois premières cuves de détente (A, B, C) étant prévues et une des autres premières cuves de détente étant vidée durant le maintien de la température dans une des premières cuves de détente (A, B, C) et une autre des premières autres cuves de détente (A, B, C) étant remplie.

6. Procédé selon l'une des revendications 4 ou 5, le condensat formé à partir des vapeurs dans le premier échangeur thermique (2) étant reconduit dans la première cuve de détente (A, B, C) se trouvant en remplissage.

7. Procédé selon l'une des revendications précédentes, la solution d'hydrolyse étant transportée dans au moins une deuxième cuve de détente (D) placée en aval des premières cuves de détente (A, B, C).

8. Procédé selon la revendication 7, d'autres vapeurs formées dans la deuxième cuve de détente (D) lors de la détente de la solution d'hydrolyse étant amenées pour préchauffer la masse résiduelle biogène via un deuxième échangeur thermique (3).

9. Procédé selon la revendication 8, l'autre condensat formé à partir des autres vapeurs dans le deuxième échangeur thermique (3) étant reconduit dans la deuxième cuve de détente (D).

10. Procédé selon l'une des revendications précédentes, la masse résiduelle biogène à l'opération mentionnée au point b) étant amenée par un réacteur à écoulement en boucles.

11. Dispositif destiné à effectuer le procédé selon l'une des revendications précédentes, comprenant
a) un moyen (1) pour broyer de la masse résiduelle biogène,
b) un moyen (4) pour chauffer les masses résiduelles biogènes à une température comprise dans une plage de 150°C à 250°C et pour appliquer une pression de 10 à 50 bar de façon à former une solution d'hydrolyse,
c) un moyen pour la sélection alternative d'une de plusieurs premières cuves de détente (A, B, C) et un moyen (12, 13a, 13b, 13c) pour transporter la solution d'hydrolyse dans la cuve de détente sélectionnée (A, B, C),
d) un moyen (D) pour refroidir et détendre la solution d'hydrolyse et
e) un moyen (10) pour transformer la solution d'hydrolyse au moyen de microorganismes.

12. Dispositif selon la revendication 11, le moyen (4) pour chauffer les masses résiduelles biogènes à une température comprise entre 150°C et 250°C et pour appliquer une pression de 10 à 50 bar comprenant un réacteur à écoulement en boucles.

13. Dispositif selon la revendication 11 ou 12, un moyen (12) étant prévu pour transporter la solution d'hydrolyse de la première (A, B, C) à une autre première cuve de détente (A, B, C).

14. Dispositif selon l'une des revendications 11 à 13, un premier échangeur thermique (2) relié aux premières cuves de détente (A, B, C) étant prévu pour préchauffer la masse résiduelle biogène.

15. Dispositif selon l'une des revendications 11 à 14, trois premières cuves de détente (A, B, C) étant prévues.

16. Dispositif selon l'une des revendications 11 a 15, au moins une deuxième cuve de détente (D) placée en aval des premières cuves de détente (A, B, C) étant prévue.

17. Dispositif selon l'une des revendications 11 à 16, la deuxième cuve de détente (D) étant reliée à un deuxième échangeur thermique (3) pour préchauffer la masse résiduelle biogène.

18. Dispositif selon l'une des revendications précédentes 11 à 17, une première conduite circulaire (5) reliant le premier échangeur thermique (2) aux premières cuves de détente (A, B, C) étant prévue pour la rétroaction du condensat formé à partir des vapeurs dans le premier échangeur thermique (2).

19. Dispositif selon l'une des revendications précédentes 11 à 18, une deuxième conduite circulaire (7) reliant le deuxième échangeur thermique (3) à la deuxième cuve de détente (D) étant prévue pour la rétroaction de l'autre condensat formé à partir des autres vapeurs dans le deuxième échangeur thermique (3).

20. Dispositif selon l'une des revendications 11 à 19, la masse résiduelle biogène mentionnée à l'opération point b) étant amenée par un réacteur à écoulement en boucles.
